# DEMANDE DE BREVET EUROPEEN

(11) **EP 0 717 998 A1**
(43) Date de publication de la demande: **26.06.1996**
(21) Numéro de dépôt: 95402570.6
(22) Date de dépôt: 16.11.1995
(51) Int. Cl.: A61K 38/08, A61K 38/10, A61K 31/135, A61K 31/41, A61K 7/02, A61K 7/48

(54) **Utilisation d'un antagoniste de substance P pour le traitement des prurits, des algies oculaires et/ou palpébrales et des dysesthésies oculaires ou palpébrales**

(30) Priorité: 19.12.1994 FR 9415255
(71) Demandeur: L'OREAL, F-75008 Paris (FR)
(72) Inventeur: De Lacharriere, Olivier, F-75015 Paris (FR); Breton, Lionel, F-78000 Versailles (FR)
(74) Mandataire: Lhoste, Catherine

(57) **Abrégé**

L'invention se rapporte à l'utilisation d'un antagoniste de substance P dans ou pour la préparation d'une composition cosmétique ou pharmaceutique notamment à application topique pour traiter les prurits, les algies oculaires ou palpébrales et les dysesthésies oculaires ou palpébrales. La composition cosmétique peut être utilisée pour le maquillage ou le démaquillage des yeux sensibles.

## Description

La présente invention se rapporte à l'utilisation d'un antagoniste de substance P pour la préparation d'une composition pharmaceutique pour traiter notamment par voie topique les prurits, les dysesthésies oculaires et/ou palpébrales, et les algies oculaires et/ou palpébrales ainsi qu'à l'utilisation d'un antagoniste de substance dans une composition cosmétique destinée au soin ou au maquillage des yeux ou des paupières.

Certains patients souffrent d'algies oculaires et/ou palpébrales à la suite d'opérations ou de coups reçus sur l'oeil. Par ailleurs, certaines personnesprésentent trés fréquemment sans qu'on n'en connaisse la cause précise des sensations de démangeaisons ou prurit et des sensations dysesthésiques au niveau des yeux et des paupières.

Par sensations dysesthésiques, on entend des sensations de brûlures ou d'échauffement, de picotements, de fourmillements, d'inconforts, de tiraillements, etc ... Ces sensations peuvent s'associer à des rougeurs sans pour autant qu'il s'agisse de conjonctivites.

L'ensemble de ces signes ophtalmiques peuvent en outre s'associer à une rosacée, indépendamment de l'existence d'une conjonctivite.

Parmi les facteurs déclenchants les crises prurigineuses ou dysesthésiques ophtalmiques ou palpébrales, on peut citer les variations de température rapide, la chaleur et notamment l'exposition aux ultraviolets ou aux infrarouges, l'humidité relative basse, l'exposition aux vents violents ou aux courants d'air (soufflerie, air conditionné), l'application de tensioactifs, l'exposition à des vapeurs toxiques ou irritantes (solvants...), les gouttes ou topiques ophtalmologiques irritants, les topiques palpébraux dermatologiques ou cosmétiques irritants (alpha-hydroxyacides...), ou l'utilisation de certains cosmétiques même lorsque ceux-ci ne sont pas connus comme particulièrement irritants.

Jusqu'alors, le mécanisme de formation de ces signes était très mal connu et les dysesthésies oculaires et/ou palpébrales étaient traitées par des corticoïdes et également des antiseptiques locaux en pommade ophtalmique ou en gouttes.

Les corticoïdes sont relativement efficaces pour calmer les symptômes ci-dessus, mais malheureusement, ils présentent des effets secondaires souvent très pénalisants comme des atrophies. De plus, ils sensibilisent aux infections mycosiques ou bactériennes et leur cinétique d'action est souvent lente (plusieurs minutes à quelques heures). Par ailleurs, leur utilisation chronique peut conduire à une pharmacodépendance.

II subsiste donc le besoin d'un traitement des prurits, des algies et des dysesthésies oculaires et palpébrales ne présentant pas les inconvénients ci-dessus.

La présente invention a justement pour objet l'utilisation d'un ou plusieurs antagonistes de substance P pour traiter ces affections.

La substance P est un élément chimique polypeptidique élaboré et libéré par une terminaison nerveuse. Elle fait partie de la famille des tachykinines qui proviennent des terminaisons nerveuses sensibles, libres de l'épiderme et du derme. La substance P intervient notamment dans la transmission de la douleur et dans des maladies du système nerveux central tels que l'anxiété, la schizophrénie, dans des maladies respiratoires et inflammatoires, dans des maladies gastro-intestinales, dans des maladies rhumatismales et dans certaines maladies dermatologiques telles que l'eczéma, le psoriasis, l'urticaire et les dermites de contact.

Il est connu d'utiliser les antagonistes de substance P pour traiter les maladies indiquées ci-dessus. A cet effet, on peut se référer aux documents US-A-4472305, US-A-4839465, EP-A-101929, EP-A-333174, EP-A-336230, EP-A-394989, EP-A-443132, EP-A-498069, EP-A-515681, EP-A-517589, WO-A-92/22569, GB-A-2216529, EP-A-360390, EP-A-429366, EP-A-430771, EP-A-499313, EP-A-514273, EP-A-514274, EP-A-514275, EP-A-514276, EP-A-520555, EP-A-528495, EP-A-532456, EP-A-545478, EP-A-558156, WO-A-90/05525, WO-A-90/05729, WO-A-91/18878, WO-A-91/18899, WO-A-92/12151 , WO-A-92/15585, WO-A-92/17449, WO-A-92/20676, WO-A-93/00330, WO-A-93/00331, WO-A-93/01159, WO-A-93/01169, W0-A-93/01170, WO-A-93/06099, WO-A-93/09116, EP-A-522808, WO-A-93/01165, WO-A-93/10073 et WO-A-94/08997.

Cependant, personne n'avait envisagé jusqu'à ce jour d'utiliser les antagonistes de substance P pour traiter les prurits et/ou les algies oculaires et/ou palpébrales et/ou les dysesthésies oculaires ou palpébrales.

Aussi, la présente invention a pour objet l'utilisation d'au moins un antagoniste de substance P pour la préparation d'une composition pharmaceutique ou dermatologique pour traiter les prurits et/ou les algies oculaires et/ou palpébrales et/ou les dysesthésies oculaires ou palpébrales.

L'application de compositions contenant un ou des antagonistes de substance P sur les yeux ou les paupières permet d'obtenir une nette diminution voire une disparition complète des algies, sensations dysesthésiques et prurits ophtalmiques ; on constate très rapidement, et en tout état de cause beaucoup plus rapidement qu'avec les corticoïdes, un effet calmant et apaisant, préventif et curatif sur les yeux et les paupières. En outre, on note aucune pharmacodépendance.

Grâce à ces antagonistes de substance P, il est en outre possible de concevoir des compositions cosmétiques pour yeux sensibles et en particulier des lotions démaquillantes ou de nettoyage des yeux, des produits de maquillage pour yeux sensibles et notamment des fards à paupières, des mascaras, des crayons ou des eye-liners pour yeux sensibles.

Aussi, l'invention à encore pour objet l'utilisation d'au moins un antagoniste de substance P dans une composition cosmétique contenant un milieu cosmétiquement acceptable, destinée aux yeux sensibles.

La composition de l'invention contient un milieu cosmétiquement, pharmaceutiquement ou dermatologiquement acceptable, c'est-à-dire un milieu compatible avec la peau, et les yeux. La composition contenant l'antagoniste de substance P est appliquée notamment par voie topique. Elle peut également être ingérée ou injectée.

Pour qu'une substance soit reconnue comme un antagoniste de substance P, elle doit répondre à la caractéristique suivante :
- avoir une activité pharmacologique antagoniste de la substance P, c'est-à-dire induire une réponse pharmacologique cohérente dans au moins l'un des deux tests suivants :
   - la substance antagoniste doit diminuer l'extravasation du plasma au travers de la paroi vasculaire induite par la capsaïcine ou par une stimulation nerveuse antidromique, ou bien
   - la substance antagoniste doit provoquer une inhibition de la contraction des muscles lisses induites par l'administration de substance P.

L'antagoniste de substance P peut en outre avoir une affinité sélective pour les récepteurs NK1 des tachykinines.

L'antagoniste de substance P de l'invention peut être fonctionnel ou réceptoriel, c'est-à-dire inhiber la synthèse et/ou la libération de substance P, ou empêcher sa fixation et/ou moduler son action.

L'antagoniste de substance P de l'invention peut être notamment un peptide ou un dérivé azoté non peptidique, et plus précisément un composé comportant un hétérocycle azoté, soufré ou oxygéné, ou un composé comprenant un atome d'azote lié directement ou indirectement à un cycle benzènique.

On peut utiliser dans l'invention par exemple comme peptide antagoniste de substance P le sendide et le spantide II.

Le sendide correspond à la formule : Tyr D-Phe Phe D-His Leu Met NH2 dans laquelle :
Tyr représente la tyrosine,
D-Phe représente la D-phénylalanine,
Phe représente la phénylalanine,
D-His représente la D-histidine,
Leu représente la leucine,
Met représente la méthionine.

Le spantide II correspond à la formule : D-NicLys Pro 3-Pal Pro D-Cl2Phe Asn D-Trp Phe D-Trp Leu Nle NH2 dans laquelle :
D-NicLys représente.le nicotinate de D-lysine,
Pro représente la proline,
3-Pal représente la 3-pyridyl-alanine,
D-Cl₂Phe représente la D-dichlorophénylalanine,
Asn représente l'asparagine,
D-Trp représente le D-tryptophane,
Phe représente la phénylalanine,
Leu représente la leucine,
Nle représente la nor-leucine.

On peut également utiliser dans l'invention comme peptide antagoniste de substance P les peptides décrits dans les documents US-A-4472305, US-A-4839465, EP-A-101929, EP-A-333174, EP-A-336230, EP-A-394989, EP-A-443132, EP-A-498069, EP-A-515681, EP-A-517589, WO-A-92/22569 et GB-A-2216529.

Les antagonistes de substance P non peptidiques utilisables dans l'invention sont notamment des composés hétérocycliques, notamment azotés, soufrés ou oxygénés, ou des composés comprenant un atome d'azote lié directement ou indirectement à un ou plusieurs cycles benzéniques.

Comme composé hétérocyclique, on peut utiliser dans l'invention ceux comportant un hétérocycle azoté décrits dans les documents suivants : EP-A-360390, EP-A-429366, EP-A-430771, EP-A-499313, EP-A-514273, EP-A-514274, EP-A-514275, EP-A-514276, EP-A-520555, EP-A- 528495, EP-A-532456, EP-A-545478, EP-A-558156, WO-A-90/05525, WO-A-90/05729, WO-A-91/18878, WO-A-91/18899, WO-A-92/12151, WO-A-92/15585, WO-A-92/17449, WO-A-92/20676, WO-A-93/00330, WO-A-93/00331, WO-A-93/01159, WO-A-93/01169, W0-A-93/01170, WO-A-93/06099, WO-A-93/09116, WO-A-94/08997. En particulier, le composé comprenant au moins un hétérocycle azoté est un dérivé de 2-tricyclyl-2-amino-éthane, un dérivé de spirolactame, un dérivé de quinuclidine, un dérivé azacyclique, un dérivé d'aminopyrrolidine, un dérivé de pipéridine, un aminoazahétérocycle, un dérivé d'isoindole.

Comme autres composés hétérocycliques, on peut citer les composés hétérocycliques oxygénés ou soufrés tels que les dérivés du furanne, les dérivés du benzofuranne, les dérivés du thiophène et les dérivés du benzothiophène, comportant éventuellement des substituants azotés, tels que les composés hétérocycliques décrits dans les documents US-A-4931459, US-A-4910317 et EP-A-299457, et plus spécialement les alcoxy- et/ou aryloxy- tétrazolylbenzofuranne-carboxamides ou les alcoxy- et/ou aryloxy- tétrazol-yl-benzothiophène-carboxamides.

Comme composés comportant un atome d'azote lié directement ou indirectement à un noyau benzénique, on peut citer ceux décrits dans les documents suivants : EP-A-522808, WO-A-93/01165 et WO-A-93/10073. On peut citer notamment les dérivés d'éthylène diamine, tels que la N,N'-bis-di-(3,5-dimethylbenzyle)- éthylène diamine, la N,N'-bis-di-(3,5-dimethoxybenzyle)-éthylène diamine ; ces composés sont décrits comme intermédiaires de synthèse dans le document WO-A-93/11338 déposé au nom de la demanderesse.

Les antagonistes de substance P peuvent être synthétisés ou extraits de produits naturels (végétaux ou animaux).

Dans les compositions selon l'invention, l'antagoniste de substance P est utilisé de préférence en une quantité allant de 0,000001 à 5 % en poids par rapport au poids total de la composition, et en particulier en une quantité allant de 0,0001 à 0,1 % en poids par rapport au poids total de la composition.

Les compositions selon l'invention peuvent se présenter sous toutes les formes galéniques normalement utilisées pour une application topique ; la composition peut se présenter notamment sous forme de solutions aqueuses, hydroalcooliques ou huileuses, ou de dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, de microémulsions, de microcapsules, de microparticules, de dispersions vésiculaires de type ionique et/ou non ionique, de poudres compactées ou coulées. Ces compositions sont préparées selon les méthodes usuelles.

Pour une application topique à visée thérapeutique, les compositions se présentent notamment sous forme de collyre, pommade, ou de solution de lavage oculaire. Pour une application cosmétique, les compositions peuvent notamment constituées des crèmes de soin ou de protection pour yeux sensibles, des laits ou lotions de nettoyage ou de démaquillage des yeux sensibles, des produits de maquillage des yeux notamment sensibles comme des crayons, des mascaras, des eye-liners, des fards à paupières.

Les compositions injectables peuvent se présenter sous forme d'une lotion aqueuse, huileuse ou sous forme de sérum.

Les compositions utilisées par voie orale peuvent se présenter sous forme de capsules, de gélules, de sirops ou de comprimés.

Les quantités des différents constituants des compositions selon l'invention sont celles classiquement utilisées dans les domaines considérés.

Lorsque la composition de l'invention est une émulsion, la proportion de la phase grasse peut aller de 5 % à 80 % en poids, et de préférence de 5 % à 50 % en poids par rapport au poids total de la composition. Les huiles, les émulsionnants et les coémulsionnants utilisés dans la composition sous forme d'émulsion sont choisis parmi ceux classiquement utilisés dans les domaines cosmétique et dermatologique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la quantité d'huile peut aller jusqu'à plus de 90 % en poids du poids total de la composition.

De façon connue, la composition de l'invention peut contenir également des adjuvants habituels dans les domaines considérés, tels que les gélifiants hydrophiles ou lipophiles, les actifs hydrophiles ou lipophiles, les conservateurs, les antioxydants, les solvants, les parfums, les charges, les filtres, les absorbeurs d'odeur, des pigments et les matières colorantes. Les quantités de ces différents adjuvants sont celles classiquement utilisées dans les domaines considérés, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles siliconées (cyclométhicone) et les huiles fluorées (perfluoropolyéthers). On peut aussi utiliser des alcools gras, des acides gras (acide stéarique) ou encore des cires (paraffine, carnauba, cire d'abeilles).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium, la silice hydrophobe, les polyéthylènes et l'éthylcellulose.

Comme actifs hydrophiles, on peut utiliser les protéines ou les hydrolysats de protéine, les acides aminés, les polyols, l'urée, l'allantoïne, les sucres et les dérivés de sucre, les vitamines, les extraits végétaux, notamment d'Aloe Vera.

Comme actifs lipophiles, on peut utiliser le tocophérol (vitamine E) et ses dérivés, le rétinol (vitamine A) et ses dérivés, les acides gras essentiels, les céramides, les huiles essentielles.

On peut également associer les antagonistes de substance P à des agents actifs, notamment des cicatrisants (par exemple vitamine B₁₂), des antiseptiques (par exemple acide borique), des antiallergiques (par exemple chromoglycate de sodium), des antiviraux (par exemple acyclovir), des anesthésiques (par exemple chlorhydrate de lidocaïne et dérivés) et des anti-inflammatoires non stéroïdiens (par exemple indométhacine).

Les exemples suivants illustrent l'invention. Dans ces exemples, les proportions indiquées sont des pourcentages en poids.

### Exemple 1 : Collyre

| | |
|---|---|
| Sendide | 0,5 % |
| Excipient : | qsp 100 % |
| Chlorure de sodium | |
| Borate de sodium | |
| Polysorbate 80 | |
| Acide borique | |
| Eau | |

### Exemple 2 : Pommade

| | |
|---|---|
| N,N'-bis-di-(3,5-diméthoxybenzyle)-éthylène diamine | 1 % |
| Excipient : | qsp 100 % |
| Chlorure de benzalkonium | |
| Edetate de sodium | |
| D-mannitol | |
| Carbomer | |
| Soude | |
| Eau | |

### Exemple 3 : Solution

| | |
|---|---|
| Spantide II | 2 % |
| Excipient : | |
| Acide borique | 5 % |
| Chlorure de sodium | 0,3 % |
| Borate de phénylmercure | 0,5 % |
| Eau | qsp 100 % |

### Exemple 4 : Pommade

Cet exemple se différencie de l'exemple 2, par l'utilisation comme d'antagoniste de substance P du 3-benzyloxy-5-méthoxy-N-III-tétrazol-5-yl-benzothiophène-2-carboxamide fabriqué selon l'exemple 1 du document EP-A-299457.

## Revendications

1. Utilisation d'au moins un antagoniste de substance P pour la préparation d'une composition pharmaceutique ou dermatologique pour traiter les prurits et/ou les algies oculaires et/ou palpébrales et/ou les dysesthésies oculaires ou palpébrales.

2. Utilisation d'au moins un antagoniste de substance P dans une composition cosmétique contenant un milieu cosmétiquement acceptable, destinée aux yeux sensibles.

3. Utilisation selon la revendication 1 ou 2, caractérisée en ce que l'antagoniste de substance P est choisi parmi les peptides, les composés comprenant au moins un hétérocycle et les composés azotés comprenant un ou plusieurs cycles benzèniques.

4. Utilisation selon la revendication 3, caractérisée en ce que le peptide est le sendide ou le spantide II.

5. Utilisation selon la revendication 3, caractérisée en ce que le composé comprenant au moins un hétérocycle est un composé hétérocyclique azoté choisi parmi les dérivés de 2-tricyclyl-2-amino-éthane, les dérivé de spirolactame, les dérivés de quinuclidine, les dérivés azacycliques, les dérivés d'aminopyrrolidine, les dérivés de pipéridine, les aminoazahétérocycles, les dérivés d'isoindole.

6. Utilisation selon la revendication 3, caractérisée en ce que le composé comprenant au moins un hétérocycle est un composé hétérocyclique oxygéné ou soufré choisi parmi les dérivés du furanne, les dérivés du benzofuranne, les dérivés du thiophène et les dérivés du benzothiophène, et notamment les tétrazolyl-benzofuranne-carboxamides ou les tétrazolyl-benzothiophène-carboxamides.

7. Utilisation selon la revendication 3, caractérisée en ce que le composé azoté comprenant au moins un cycle benzènique est un dérivé d'éthylène diamine.

8. Utilisation selon l'une quelconque des revendications précédentes, caractérisée en ce que l'antagoniste de substance P est utilisé en une quantité allant de 0,000001 à 5 % en poids par rapport au poids total de la composition.

9. Utilisation selon l'une quelconque des revendications précédentes, caractérisée en ce que l'antagoniste de substance P est utilisé en une quantité allant de 0,0001 à 0,1 % en poids par rapport au poids total de la composition.

10. Utilisation selon l'une quelconque des revendications précédentes, caractérisée en ce que la composition contient en outre au moins un actif choisi parmi les protéines ou les hydrolysats de protéine, les acides aminés, les polyols, l'urée, les sucres et les dérivés de sucre, les vitamines, l'amidon, les extraits végétaux, les céramides, les huiles essentielles.

11. Utilisation selon l'une quelconque des revendications précédentes, caractérisée en ce que la composition contient en outre au moins un agent choisi parmi les agents cicatrisants, antiseptiques, antiallergiques, anesthésiques, antiviraux, anti-inflammatoires non stéroïdiens.

12. Utilisation selon l'une quelconque des revendications précédentes, caractérisée en ce que la composition se présente sous une forme appropriée à une application topique, injectable ou ingérable.

13. Utilisation selon l'une quelconque des revendications précédentes, caractérisée en ce que la composition est une solution aqueuse, huileuse ou hydroalcoolique, une émulsion eau-dans-huile, une émulsion huile-dans-eau, une microémulsion, un gel aqueux, un gel anhydre, un sérum, une dispersion de vésicules, de microcapsules ou de microparticules, une poudre compactée ou coulée.

14. Utilisation selon l'une quelconque des revendications précédentes, caractérisée en ce que la composition est destinée au maquillage ou au démaquillage des yeux.

15. Utilisation selon l'une quelconque des revendications précédentes, caractérisée en ce que la composition contient en outre au moins un adjuvant choisi parmi les émulsionnants, les conservateurs, les antioxydants, les solvants.
